# EUROPÄISCHE PATENTSCHRIFT

(11) **EP 3 665 265 B1**
(45) Veröffentlichungstag und Bekanntmachung des Hinweises auf die Patenterteilung: **28.10.2026**
(21) Anmeldenummer: 18742758.8
(22) Anmeldetag: 13.07.2018
(51) Int. Cl.: C12M 3/00, C12M 1/00, C12M 1/12, C12M 1/34

(54) **VORRICHTUNG ZUR KULTIVIERUNG VON GEWEBESCHNITTEN**
DEVICE FOR CULTIVATING TISSUE SECTIONS
DISPOSITIF POUR LA CULTURE DE COUPES TISSULAIRES

(30) Priorität: 10.08.2017 DE 102017213923
(43) Veröffentlichungstag der Anmeldung: 17.06.2020
(73) Patentinhaber: Robert Bosch Gesellschaft für medizinische Forschung mbH, 70376 Stuttgart (DE)
(72) Erfinder: DONG, Meng, 70437 Stuttgart (DE); SCHWAB, Matthias, 70176 Stuttgart (DE); VAN DER KUIP, Heiko, 72108 Rottenburg (DE)
(74) Vertreter: Bee, Joachim
(86) Internationale Anmeldenummer: PCT/EP2018/069067
(87) Internationale Veröffentlichungsnummer: WO 2019/029947

(56) Entgegenhaltungen:
- EP-A1- 1 260 265
- EP-A1- 1 857 543
- EP-A1- 2 230 297
- EP-A1- 2 420 569
- EP-A1- 3 147 349
- EP-B1- 0 842 262
- WO-A2-2006/134432
- DE-A1- 102013 203 306
- DE-B3- 102014 222 547
- US-A1- 2007 048 732
- US-A1- 2010 047 853
- US-A1- 2011 287 982
- UNIVERSITÄT LEIPZIG: "Meilenstein in der Langzeitkultivierung von Gewebe gesetzt", 25 April 2012 (2012-04-25), pages 1 - 3, XP002785945, Retrieved from the Internet <URL:https://www.bionity.com/de/news/137654/meilenstein-in-der-langzeitkultivierung-von-gewebe-gesetzt.html> [retrieved on 20181019]
- DATABASE WPI Week 199804, Derwent World Patents Index; AN 1998-038286, XP002785946
- JOHN A. HICKMAN ET AL: "Three-dimensional models of cancer for pharmacology and cancer cell biology: Capturing tumor complexity in vitro/ex vivo", BIOTECHNOLOGY JOURNAL, vol. 9, no. 9, 30 September 2014 (2014-09-30), DE, pages 1115 - 1128, XP055251805, ISSN: 1860-6768, DOI: 10.1002/biot.201300492

## Beschreibung

Die vorliegende Erfindung betrifft eine Vorrichtung zur Kultivierung von Gewebeschnitten, insbesondere Tumor-Gewebeschnitte, für die Medikamententestung.

### Stand der Technik

In vielen Bereichen der wissenschaftlichen Forschung sowie der Diagnostik und in präklinischen Versuchsansätzen besteht ein großer Bedarf an Modellsystemen komplexer Zellanordnungen, anhand derer bspw. die Toxizität und/oder der Metabolismus von Wirkstoffen bzw. Medikamenten *in vitro* getestet werden können. Insbesondere bei der Testung von Wirkstoffen auf Tumoren sind reproduzierbare Modellsysteme äußerst wichtig, da mehr als 90 % der in präklinischen *in vitro-* und *in* vivo-Modellen erfolgreich getesteten Antitumor-Medikamente in klinischen Studien versagen, sowie ca. 66 % aufgrund fehlender Wirksamkeit im menschlichen Körper nicht therapeutisch einsetzbar sind (Arrowsmith (2011) "Trial watch: Phase III and submission failures: 2007-2010". Nature Reviews. Drug discovery, 10, 87). Diese enorme Fehlerrate belegt eindrücklich, dass die momentan verwendeten Modellsysteme, die zur Medikamententestung verwendet werden, die Situation von Tumoren in Patienten nicht ausreichend widerspiegeln.

Bei menschlichen soliden Tumoren handelt es sich nicht nur um eine Anhäufung autonomer Tumorzellen, sondern vielmehr um komplexe organähnliche Gewebe aus Tumorzellen und Stromazellen (Tumor-assoziierte Fibroblasten und inflammatorische Zellen), die eingebettet in eine extrazelluläre Matrix sind und durch ein endotheliales Gefäßsystem versorgt werden. Die zelluläre Mikroumgebung einer Tumorzelle ist somit ein integraler und essenzieller Bestandteil eines Tumors und spielt eine zentrale Rolle bei der Tumorgenese (Bissell, M.J. et al. (2001) "Putting tumors in context". Nature Reviews. Cancer, 1, 46-54), der Tumorprogression und Metastasierung (Roskelly et al. (2002) "The dominance of the microenvironment in breast ovarian cancer". Seminars in Cancer Biology, 12, 97-104) sowie der Sensitivität gegenüber Antitumor-Medikamenten (Östmann (2012) "The tumor microenvironment controls drug sensitivity". Nature Medicine, 18, 1332-1334).

Die Wirkung eines Antitumormedikaments auf den Gesamttumor wird damit nicht nur von der intrinsischen Sensitivität der Tumorzelle selbst, sondern auch durch die zelluläre Mikroumgebung der Tumorzelle entscheidend beeinflusst.

US 2007048732 offenbart ein Kultivierungssystem mit einer Vorrichtung zur Kultivierung von Gewebeschichten zur Testung von Wirkstoffen auf bioartifizielle Organe in Form eines Gewebeschnitts.

Die Entwicklung neuer präklinischer Modelle, die diese komplexe Situation im Tumor besser widerspiegeln können als die im Moment verfügbaren, ist für die Forschung, Diagnostik und insbesondere Pharmaindustrie, aber auch für eine individuelle Sensitivitätstestung (individualisierte Tumortherapie) von herausragendem Interesse.

Mit der direkten Kultivierung von aus OP-Material gewonnenen präzise geschnittenen Tumorgewebsscheiben kann das individuelle Tumorgewebe *ex vivo* weitgehend erhalten werden (Hickman et al. (2014) "Three-dimensional models of cancer for pharmacology and cancer cell biology: capturing tumor complexity in vitro/ex vivo." Biotechnology Journal, 9, 1115-1128; van der Kuip et al. (2016) "Short term culture of breast cancer tissues to study the activity of the anticancer drug taxol in an intact tumor environment." BMC Cancer, 6, 86), ganz im Gegensatz zu allen bislang verfügbaren präklinischen Modellen.

Zur Kultivierung solcher Tumorschnitte sind verschiedene Verfahren bekannt und erfolgreich etabliert, wobei allen bislang verfügbaren Methoden gemeinsam ist, dass diese Kultivierung nur eine sehr begrenzte Zeit möglich ist und es nach wenigen Tagen zum Absterben der Tumorzelle kommt. Auf Basis herkömmlicher Kultivierungstechniken werden somit Medikamente an *per se* sterbendem Gewebe getestet, was die Verlässlichkeit dieses Systems als präklinisches Modell wiederum in Frage stellt. Diese zeitlich sehr begrenzte Kultivierungsmöglichkeit ist auf die Tatsache zurückzuführen, dass es bei Entnahme des Tumors zwangsläufig zu einer kompletten Unterbindung der Blutversorgung kommt und die Versorgung der *ex vivo* in Medium kultivierten Tumorgewebe mit physiologischen Konzentrationen an Sauerstoff sehr schwierig ist. Dies liegt an der unterschiedlichen Aufnahmekapazität von Sauerstoff in Kulturmedium im Vergleich zu Blut. So enthält sauerstoffgesättigtes Wasser unter normalen atmosphärischen Bedingungen bei einem Druck von 1 atm und einer Temperatur von 25°C 0,28 mmol/L Sauerstoff (O₂) (Carpenter (1966) "New measurements of oxygen solubility in pure and natural water". Limnol Oceanogr. , 11 (2): 264-277), wobei dieser Wert in Medium aufgrund des Salzgehalts sogar noch etwas niedriger ist. Im Blut wird Sauerstoff vorwiegend (zu etwa 98%) in den Erythrozyten reversibel an Hämoglobin (Hb) gebunden transportiert. Hämoglobin wird in den Kapillaren der Lunge mit bis zu 1,34 ml/g Sauerstoff beladen und kann dann in den Kapillargebieten der Organe und Gewebe des Körpers den Sauerstoff wieder abgeben. Der Gesamtsauerstoffgehalt im arteriellen Blut ist mit 8,47 mmol/L (bei einem Partialdruck von 100 mmHg) vergleichbar mit dem der Luft (8,6 mmol/L bei einem Partialdruck von 159 mmHg). Selbst im venösen Blut liegt der Sauerstoffgehalt noch bei 5,3 bis 6,2 mmol/L (bei einem Partialdruck von 40 mmHg; Martin (1999) "All You Really Need to Know to Interpret Arterial Blood Gase" LWW; Second edition, ISBN-10: 0683306049). Somit stellt Blut aufgrund dieses Gastrasportmechanismus etwa 10 mal mehr Sauerstoff zur Verfügung als sauerstoffgesättigtes Medium.

Im Gewebe hängt die Sauerstoffaufnahme *in vivo* vom dort vorherrschenden Partialdruck ab: Ist dieser hoch, wie beispielsweise in der Lunge, so wird Sauerstoff ins Blut aufgenommen. In den meisten Tumorgeweben ist der Sauerstoffpartialdruck sehr niedrig, sodass hier besonders viel Sauerstoff aus dem Blut ins Gewebe abgegeben wird. Dies kann sauerstoffgesättigtes Medium nicht leisten. Nach Unterbindung der Blutversorgung kommt es somit in Tumorgeweben in einer konventionellen Flüssigkeitskultur zu einer akuten Unterversorgung mit Sauerstoff, einer Reduktion der Zellatmung und damit einhergehend zu Zellstress und letztendlich Zelltod.

Zwar können die bekannten Systeme mit Kultivierungsvorrichtungen für Gewebe an der Luft-Flüssigkeits-Grenzschicht (air-liquid-interface) diesen Mangel teilweise beheben, indem eine Gewebeseite an Luft exponiert wird, wodurch diese Art der Kultivierung von Gewebeschnitten der Flüssigkultur klar überlegen ist, doch führt diese zur Ausbildung eines artifiziellen Gradienten, der von der Luft- zur Mediumseite orientiert ist, und zwar derart, dass nur die oberen, der Luft zugewandten Zellschichten dabei in ihrer Morphologie und Beschaffenheit dem Originalgewebe, aus dem die Tumorschnitte gewonnen wurden, entsprechen, während die der Luft abgewandten Zellschichten Zeichen von Hypoxie und Nekrose aufweisen.

### Offenbarung der Erfindung

Vor diesem Hintergrund ist es Aufgabe der vorliegenden Erfindung, eine Vorrichtung bereitzustellen, mit der die vorstehend genannten Nachteile aus dem Stand der Technik vermindert oder gänzlich vermieden werden können.

Erfindungsgemäß werden diese und andere Ziele erreicht durch eine Vorrichtung zur Kultivierung von Gewebeschnitten, insbesondere für die Testung von Wirkstoffen und/oder Medikamenten, wobei die Vorrichtung Folgendes aufweist: einen Rahmen mit einem umlaufenden Profil und einen vom umlaufenden Profil gebildeten mittigen Freiraum, zumindest eine Trägerkomponente, wobei die Trägerkomponente zur Aufnahme eines Gewebeschnittes ausgestaltet ist, und zumindest ein Flüssigkeits-absorbierendes Streifenelement, wobei der Rahmen zum Halten der zumindest einen Trägerkomponente und des zumindest einen Streifenelements ausgestaltet ist, und zwar derart, dass das zumindest eine Streifenelement im Bereich des umlaufenden Profils angebracht ist, und dass die zumindest eine Trägerkomponente den Freiraum überspannend und das zumindest eine Streifenelement überlappend am Rahmen gehalten wird.

Ferner werden diese und andere Ziele erreicht durch ein Kultivierungssystem zur Kultivierung von Gewebeschnitten, wobei das Kultivierungssystem eine erfindungsgemäße Vorrichtung und ein Aufnahmegefäß aufweist, in das die Vorrichtung zur Kultivierung von Gewebeschnitten und ggf. Testung von Wirkstoffen und/oder Medikamenten aufgenommen werden kann.

Die der Erfindung zugrunde liegende Aufgabe wird auf diese Weise vollkommen gelöst.

Mit der erfindungsgemäßen Vorrichtung wird erreicht, dass der Gewebeschnitt beidseits mit optimalen Sauerstoffkonzentrationen versorgt wird, wodurch eine atmosphärische Kultivierung des Gewebeschnitts ermöglicht wird. Ferner kann mit der erfindungsgemäßen Vorrichtung eine konstante Perfusion mit Medium ermöglicht werden, das an beiden Seiten des Gewebeschnitts vorbeigeführt werden kann, wodurch die Versorgung mit Nährstoffen sowie eine gleichmäßige Applikation von Medikamenten gewährleistet wird.

Die erfindungsgemäße Vorrichtung erlaubt die Testung von Wirkstoffen und Medikamenten unter Beibehaltung des nativen individuellen Gewebes, bspw. eines Tumorgewebes, unter weitgehend physiologischen Bedingungen. Auch kann die Gesamtmenge an Perfusat und damit der Verbrauch an dem zu testenden Wirkstoff gering gehalten werden.

Dabei wird vorliegend unter einem "umlaufenden Profil und einem vom umlaufenden Profil gebildeten mittigen Freiraum" jede Einfassung verstanden, die zum Halten bzw. Stabilisieren einer oder mehreren in den (umlaufenden) Rahmen gespannten oder aufgenommenen Trägerkomponente geeignet ist.

Ferner wird vorliegend unter einem Flüssigkeits-absorbierenden Streifenelement jedes Element verstanden, das aus einem (Flüssigkeits-)saugfähigen Material besteht oder ein solches aufweist, und das eine Flüssigkeit aufnehmen und ggf. weiterleiten kann.

Gemäß einer bevorzugten Ausführungsform der erfindungsgemäßen Vorrichtung umfasst der Rahmen zwei Rahmen-Bauteile mit jeweils einem umlaufenden Bauteil-Profil und einem vom umlaufenden Bauteil-Profil gebildeten jeweils mittigen Bauteil-Freiraum. Beide Rahmen-Bauteile sind mittels eines Verbindungselements deckend zum Rahmen zusammensetzbar, wobei das Verbindungselement vorzugsweise als Klickverbindung, Führungsschiene, Klemmverbindung oder als Verschluss ausgestaltet ist.

Diese Ausführungsform bietet den Vorteil, dass die zumindest eine Trägerkomponente sowie das zumindest eine Streifenelement einfach zwischen die beiden Rahmen-Bauteile gegeben werden können, die durch Zusammenklippen oder stecken der beiden Rahmen-Bauteile zwischen diesen verklemmt werden können.

Der Rahmen, bzw. die beiden Rahmen-Bauteile, ist/sind dabei bevorzugt insgesamt im Wesentlichen rechteckig bzw. schmal quaderförmig und weist/weisen entsprechend vier Seiten auf.

Gemäß einer weiteren Ausführungsform weist der Rahmen eine Führung zur Flüssigkeitszufuhr auf.

Diese Führung wird gemäß einer bevorzugten Ausführungsform durch das Verbinden bzw. Zusammenfügen der beiden Bauteile des Rahmens gebildet. Alternativ befindet sich die Führung an einem der beiden Bauteile.

Vorteilhafterweise kann über die Führung eine Flüssigkeit, bspw. Medium oder eine einen zu testenden Wirkstoff oder ein zu testendes Medikament aufweisende Flüssigkeit der Vorrichtung - und damit dem in der Vorrichtung zu kultivierenden Gewebeschnitt - zugeführt und das Gewebe damit mit der Flüssigkeit perfundiert werden. Die Flüssigkeit wird dabei vom Streifenelement aufgenommen und an die dieses bedeckende Trägerkomponente weitergeleitet, wo die Flüssigkeit mit dem darin gehaltenen Gewebe in Kontakt kommt.

Gemäß einer bevorzugten Ausführungsform der erfindungsgemäßen Vorrichtung weist der Rahmen und/oder die Rahmen-Bauteile Mittel zur Befestigung des Streifenelements auf. Diese Mittel können bspw. schlitzförmige Aussparungen in dem Bauteil-Profil bzw. in den Seiten der Rahmen-Bauteile sein, in welche das zumindest eine Streifenelement eingeführt und gehalten wird.

Die Rahmen-Bauteile - bzw. gemäß einer anderen Ausführungsform lediglich ein Rahmen-Bauteil - können dabei gemäß einer bevorzugten Ausführungsform an einer ihrer Seiten jeweils ein Mittel zum Aufhängen der Vorrichtung aufweisen, über welche bspw. der Rahmen dann im zusammengesetzten Zustand in ein Aufnahmegefäß eingehängt werden kann.

Das Aufhängeelement kann dabei bspw. jeweils ein nach außen weisender Bügel sein.

Bei der erfindungsgemäßen Vorrichtung kann ferner vorgesehen sein, dass das mindestens eine Streifenelement als blattförmiges Fließpapier ausgestaltet ist.

Gemäß einer weiteren Ausführungsform ist die zumindest eine Trägerkomponente aus einem Material ausgestaltet, das ausgewählt ist aus der Gruppe bestehend aus Baumwollgewebe, Nylongewebe, Polycarbonat, Zellulose-Hydrogel, Tierdarm, insbesondere Schweinedarm, 3D-gedruckte Biomaterialien, insbesondere 3D-gedrucktes Alginatgewebe oder 3D-gedrucktes Collagengewebe.

Vorzugsweise ist die zumindest eine Trägerkomponente ein gitterförmiges und sauerstoffdurchlässiges Gewebe bzw. weist ein solches auf.

In einer bevorzugten Ausführungsform ist der Rahmen dabei aus einem lösungsmittelbeständigen Material ausgestaltet.

Gemäß einer bevorzugten Ausführungsform der erfindungsgemäßen Vorrichtung ist der Rahmen aus zwei Rahmen-Bauteilen gebildet, und jedes der zwei Rahmen-Bauteile weist jeweils zwei Streifenelemente auf, die jeweils an gegenüber liegenden Seiten eines Bauteil-Profils angebracht sind, sowie jeweils eine Trägerkomponente, die den jeweiligen Bauteil-Freiraum überspannend und die zwei Streifenelemente überlappend an jeweils einem Rahmen-Bauteil gehalten wird.

Diese Ausführungsform hat den Vorteil, dass das zu untersuchende bzw. zu kultivierende Gewebe, bspw. ein dünner Gewebeschnitt, und insbesondere ein Tumor-Gewebeschnitt, zwischen den beiden Trägerkomponenten gehalten werden kann, wenn die beiden Rahmen-Bauteile zum Rahmen zusammengeklippt bzw. -gesteckt werden. Vorzugswiese beträgt der Abstand der beiden Rahmen-Bauteile im zusammengesetzten Zustand des Rahmens dabei ca. 0,8 mm bis ca. 2,2 mm, insbesondere ca. 1,0 bis ca. 2,0 und bevorzugt ca. 1,6 mm. Dies ermöglicht es, jeweils die Streifenelemente einzusetzen, vorzugsweise an gegenüber liegenden Seiten eines rechteckigen bzw. quaderförmigen Rahmen-Bauteils und insbesondere bei einem vertikalen Aufstellen/Aufhängen der Vorrichtung an der oberen und unteren Seite.

Das Streifenelement, bzw. das Fließpapier, weist dabei bevorzugt eine Stärke von ca. 0,4 bis ca. 1,2 mm, insbesondere von ca. 0,8 mm auf.

Das Streifenelement ist vorzugsweise steril und autoklavierbar.

Erfindungsgemäß befindet sich zumindest eine Trägerkomponente zwischen den an den Bauteil-Profilen angebrachten Streifenelementen, wobei die Trägerkomponente den Freiraum überspannt.

Gemäß einer weiteren Ausführungsform weist das umlaufende Profil des ersten Rahmen-Bauteils und das umlaufenden Profil des zweiten Rahmen-Bauteils Mittel zum Verbinden, bspw. Steckverbinden, der beiden Bauteile auf. Gemäß einer bevorzugten Ausführungsform weist dabei das erste Rahmen-Bauteil längliche Aussparungen in sich gegenüber liegenden Seiten des umlaufenden Profils auf, die derart dimensioniert sind, dass darin längliche Erhebungen des zweiten Rahmen-Bauteils aufgenommen bzw. eingesteckt werden können, welche auf gegenüber liegenden Seiten des umlaufenden Profils des zweiten Rahmen-Bauteils vorgesehen sind. Damit wird erreicht, dass durch ein Zusammen- bzw. Aufeinanderlegen der beiden Rahmen-Bauteile die Mittel zum Verbinden jeweils ineinander greifen und die beiden Rahmen-Bauteile dadurch zusammengesteckt und fixiert werden können.

Wie bereits weiter oben erwähnt, betrifft die vorliegende Erfindung auch ein Kultivierungssystem zur Kultivierung von Gewebeschnitten, insbesondere für die Testung von Wirkstoffen und/oder Medikamenten, wobei das Kultivierungssystem eine wie oben beschriebene erfindungsgemäße Vorrichtung sowie ein Aufnahmegefäß aufweist, wobei die Vorrichtung in das Aufnahmegefäß, vorzugswiese vertikal hängend oder stehend, einbringbar ist.

Diese Ausführungsform hat den Vorteil, dass bspw. ein herkömmliches Gefäß, insbesondere ein belüftbares Röhrchen, bspw. ein entsprechend dimensioniertes Zentrifugenröhrchen, als Aufnahmegefäß eingesetzt werden kann, in welches die erfindungsgemäße Vorrichtung, bspw. über die Mittel zum Aufhängen der Vorrichtung, eingehängt werden kann. Das Röhrchen kann dabei eine Schraubkappe aufweisen, die einen sterilen Gasaustausch ermöglicht sowie einen Zugang zur Führung zur Flüssigkeitszufuhr erlaubt, bspw. durch einen Septum-Schlitz. Die Verschließbarkeit des Röhrchens mit der Schraubkappe ist dabei nicht beeinträchtigt.

Das Röhrchen kann gemäß einer bevorzugten Ausführungsform ausgewählt sein aus einem Röhrchen, das zwischen 10 und 100 ml fasst, und insbesondere einem 10-, 15-, 20-, 30-, 40-, 50-, 60-, 70-, 80-, 90-, oder 100-ml-Röhrchen.

Bevorzugte Maße für den Rahmen betragen zwischen ca. 18 mm bis ca. 28 mm x 21 mm bis ca. 31 mm, insbesondere ca. 23 x ca. 26 mm, sowie für den Freiraum ca. 9 mm bis ca. 19 mm x ca. 10 mm bis ca. 20 mm, insbesondere ca. 14 x ca. 15 mm.

Mit diesem erfindungsgemäßen System wird vorteilhafterweise eine vertikale Orientierung der erfindungsgemäßen Vorrichtung in dem Aufnahmegefäß erreicht und damit auch eine vertikale Orientierung des Gewebes/Gewebeschnitts in einem geschlossenen, sterilbelüftbaren Aufnahmegefäß. Das System kann dann bspw. in einem Brutschrank unter kontrollierter Atmosphäre gehalten werden.

Bei einer vertikalen Orientierung ist ferner die Vorrichtung bevorzugt derart in dem Aufnahmegefäß aufgenommen, dass an der oberen Seite des Rahmens eine Führung zur Flüssigkeitszufuhr, bspw. mittels einer Perfusionsnadel, liegt, die ggf. mit einem Perfusionsschlauch verbunden wird. Dieser kann gemäß einer bevorzugten Ausführungsform mit einer Spritze verbunden sein und unter Kontrolle einer Spritzenpumpe stehen, über welche eine kontrollierte Perfusion möglich ist. Die über dieses System eingeführte Flüssigkeit kann in dem Aufnahmegefäß nach Durchlaufen der Trägerkomponente und damit auch des Gewebes unten im Aufnahmegefäß aufgefangen werden.

Die vorliegende Erfindung betrifft damit auch die Verwendung der wie oben beschriebenen erfindungsgemäßen Vorrichtung oder des beschriebenen erfindungsgemäßen Kultivierungssystems zur Kultivierung von Geweben und/oder Gewebeschnitten für die Testung von Wirkstoffen und/oder Medikamenten.

Mit der vorliegenden Erfindung wird entsprechend und insgesamt eine einfach zu handhabende modulare Vorrichtung bereitgestellt, die es ermöglicht, präzise geschnittene (Tumor-)gewebe unter Mikroperfusion direkt in einer sehr dünnen Schicht Nährmedium zu kultivieren und einer definiert zusammengesetzten Gasatmosphäre auszusetzen. Mit der vorliegenden Erfindung können damit sowohl physiologisch relevante Sauerstoffkonzentrationen, als auch physiologisch relevante Perfusionsgeschwindigkeiten während der Kultivierung bereitgestellt werden.

Auch ist es mit der vorliegenden erfindungsgemäßen Vorrichtung und dem erfindungsgemäßen Kultivierungssystem möglich, Medikamente an individuellen Tumoren *ex vivo* unter Beibehaltung physiologisch relevanter Charakteristika, wie weitgehend intakter Mikroumgebung und angepasster Sauerstoffkonzentration, zu testen.

Im Vergleich zu herkömmlichen präklinischen *in vitro-* und *in vivo*-Modellen aber auch bereits angewandten Gewebekulturen können mit Hilfe der erfindungsgemäßen Vorrichtung Bedingungen bereitgestellt werden, die es ermöglichen, die Sensitivität und Resistenz gegenüber Medikamenten bei individuellen Patiententumoren sehr viel präziser zu ermitteln.

Erfindungsgemäß können in die Trägerkomponente Gewebe bzw. Gewebeschnitte aufgenommen werden, insbesondere Tumor-Gewebeschnitte, insbesondere von Tumoren eines Menschen. Der Tumor kann dabei ein gutartiger oder bösartiger Tumor eines beliebigen Gewebes sein, insbesondere ein solider Tumor, welcher insbesondere bzw. bspw. ausgewählt ist aus der Gruppe umfassend Tumore der Atmungsorgane, Tumore der Verdauungsorane, Brustkrebs, Ovarialkarzinom, Prostatakarzinom, malignes Melanom.

Gemäß einer Ausführungsform ist das Gewebe ausgewählt aus einem Gewebe aus Lunge, Kehlkopf, Brust, Magen, Pankreas, Prostata, Blase, Ovarium, Haut, Niere, Sinus, Dickdarm, Darm, Rektum, Speiseröhre, Herz, Milz, Gehirn und dessen Abdeckungen, Rückenmark und dessen Abdeckungen, Muskel, Bindegewebe, Nebenniere, Parathyroid, Thyroid, Gebärmutter, Hoden, Hypophyse, Fortpflanzungsorganen, Leber, Gallenblase, Auge, Ohr, Nase, Hals, Mandeln, Mund und Lymphknoten.

Die vorliegende Erfindung ist in ihrer Anwendung nicht auf die Details der Erstellung und Anordnung der Komponenten, die in der Beschreibung dargestellt oder in den Zeichnungen veranschaulicht sind, beschränkt. Die Erfindung ermöglicht andere Ausbildungsformen und kann auf verschiedene Art und Weise durchgeführt werden. Auch dient die hier verwendete Ausdrucksweise und Terminologie Beschreibungszwecken und ist nicht als einschränkend zu betrachten. Die Verwendung von "einschließlich", "umfassend" oder "aufweisend", "enthaltend", "beinhaltend" und Variationen davon soll im vorliegenden Text das danach Aufgezählte sowie Äquivalente davon und Zusätzliches mit umfassen.

Die vorliegende Erfindung wird durch die folgenden Beispiele, die keinesfalls als weiter einschränkend aufzufassen sind, weiter veranschaulicht. Der gesamte Inhalt aller in der vorliegenden Anmeldung zitierten Angaben (einschließlich Literaturstellen, ausgegebener Patente, veröffentlichter Patentanmeldungen und mitanhängiger Patentanmeldungen) wird hiermit ausdrücklich durch Bezugnahme in den vorliegenden Text aufgenommen. Im Falle eines Konfliktes ist die vorliegende Beschreibung, einschließlich beliebiger Definitionen hierin, vorrangig.

Es folgt die Beschreibung der Figuren.
Fig. 1A bis 1D zeigen die einzelnen Bestandteile einer beispielhaften Ausführungsform der erfindungsgemäßen Vorrichtung;
Fig. 2A bis 2D zeigen die einzelnen Bestandteile der erfindungsgemäßen Vorrichtung und deren schrittweises Zusammenfügen zur erfindungsgemäßen Vorrichtung;
Fig. 3A bis 3C zeigen die einzelnen Bestandteile des erfindungsgemäßen Kultivierungssystems zur Kultivierung von Gewebeschnitten;
Fig. 4A bis 4C zeigen die schrittweise Vorbereitung zur Benutzung der erfindungsgemäßen Vorrichtung in einer Gewebe-Einbettkassette;
Fig. 5 zeigt einen Längsschnitt der erfindungsgemäßen Vorrichtung;
Fig. 6 zeigt Aufnahmen einer Östrogen-Rezeptor- bzw. HIF1-alpha-Immunfärbung von Original-Tumor-Gewebeschnitten (A, D), Tumor-Gewebeschnitten, die einmal mit einer erfindungsgemäßen Vorrichtung kultiviert wurden ((B, E), und Tumor-Gewebeschnitte, die mit einer herkömmlichen Kultivierungsvorrichtung kultiviert wurden (C, F);
Fig. 7 zeigt Aufnahmen einer Östrogen-Rezeptor-Immunfärbung eines Original-Tumor-Gewebeschnitts (links) im Vergleich zu einem mit dem erfindungsgemäßen System über 7 Tage kultivierten Tumor-Gewebeschnitts (rechts); und
Fig. 8 zeigt Aufnahmen einer HIF1-alpha-Immunfärbung eines Original-Lungen-Tumor-Gewebeschnitts (links) im Vergleich zu einmal mit dem erfindungsgemäßen System kultivierten (Mitte) und einmal mit einem herkömmlichen Filtersystem (rechts) kultivierten Lungen-Tumor-Gewebeschnitten.

### Beispielhafte Ausführungsformen der Erfindung

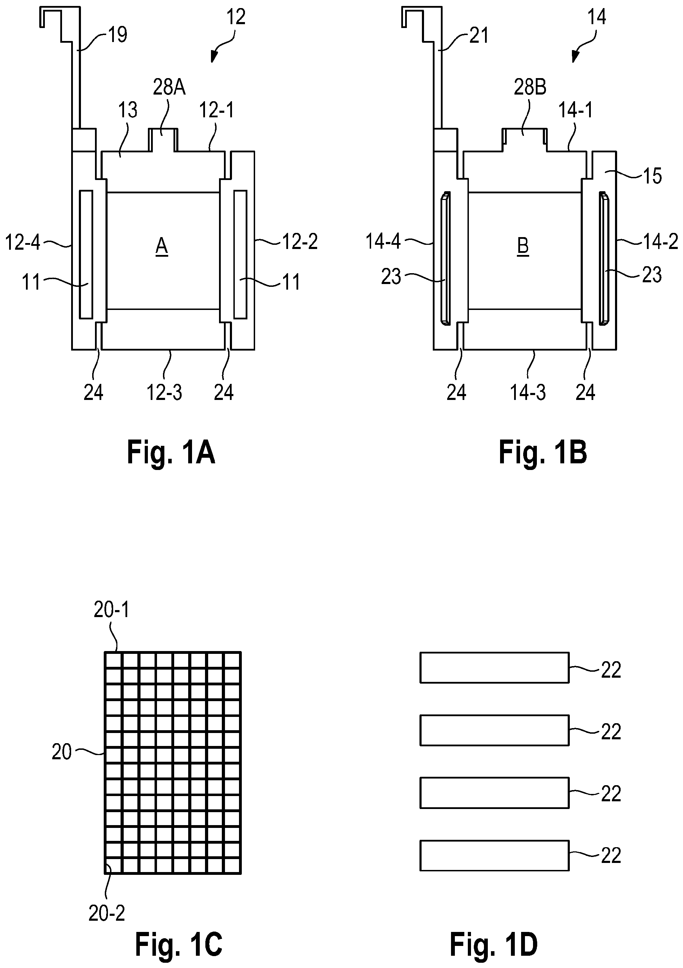

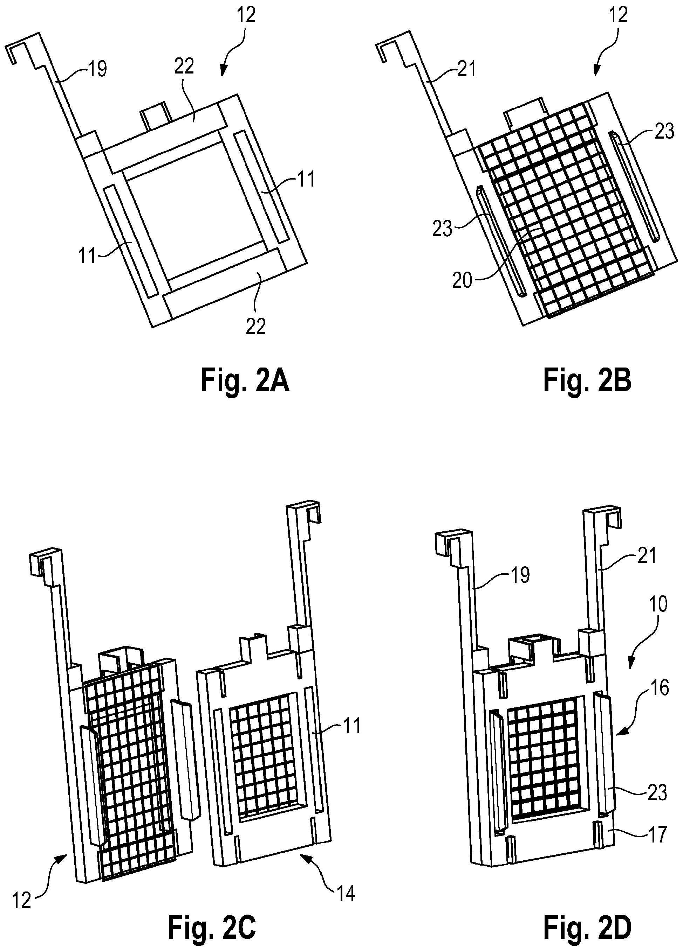

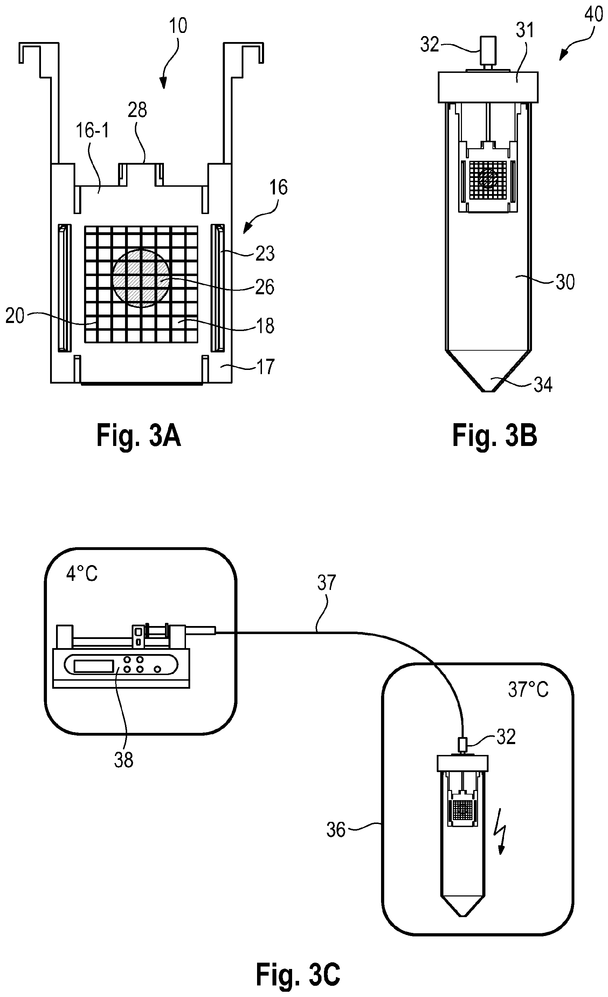

In den Figuren ist mit dem Bezugszeichen 10 insgesamt eine Ausführungsform einer erfindungsgemäßen Vorrichtung zur Kultivierung von Gewebeschnitten gezeigt. Dabei sind in Fig. 1A und 1B zwei Rahmen-Bauteile 12 und 14 gezeigt, durch deren Zusammensetzen der Rahmen 16 gebildet wird (siehe bspw. Fig. 2D und 3A).

Der Rahmen 16 weist ein umlaufendes Profil 17 sowie ein vom umlaufenden Profil 17 gebildeter Freiraum 18 auf, der in Fig. 2D und 3A von einer Trägerkomponente 20 überspannt wird.

Jedes der Rahmen-Bauteile 12 und 14 weist eine im Wesentlichen rechteckige bzw. flach quaderförmige Form mit einem umlaufenden Profil 13 bzw. 15 auf sowie mit jeweils vier Seiten 12-1, 12-2, 12-3, 12-4 bzw. 14-1, 14-2, 14-3, 14-4. Mittig befinden sich jeweils die Freiräume A und B, die durch Zusammenfügen der beiden Rahmen-Bauteile 12 und 14 den Freiraum 18 des Rahmens 16 bilden.

Beide Rahmen-Bauteile 12 und 14 weisen ferner an der Ecke der Seite 12-1 bzw. 14-1 ein Mittel zum Aufhängen 19 und 21 der Vorrichtung 10 auf.

In Fig. 1C ist eine Trägerkomponente 20 in isolierter Form gezeigt sowie in Fig. 1D vier Streifenelemente 22.

Den Figuren ist ferner zu entnehmen, dass das erste Rahmen-Bauteil 12 im umlaufenden Profil 13 auf zwei sich gegenüber liegenden Seiten 12-4 und 12-2 jeweils Aussparungen 11 aufweisen, die zur Aufnahme, bspw. durch Zusammenstecken, von der Form der Aussaprungen 11 angepassten schlitzförmigen Erhebungen 23 ausgebildet sind. Diese schlitzförmigen Erhebungen 23 befinden sich im umlaufenden Profil 15 des zweiten Rahmen-Bauteils 14 auf sich gegenüber liegenden Seiten 14-4 und 14-2.

Die Streifenelemente 22 werden an gegenüber liegenden Seiten 12-1 und 12-3 bzw. 14-1 und 14-3 der Rahmen-Bauteile 12, 14 (in Fig. 2A und 2B für das Rahmen-Bauteil 12 gezeigt) am Bauteil-Profil 13 bzw. 15 angebracht, und zwar über an den Rahmen-Bauteilen 12, 14 vorgesehene Mittel zur Befestigung 24, die in dem in den Figuren gezeigten Beispiel Schlitze im Bauteil-Profil an gegenüber liegenden Seiten 12-1, 12-3 bzw. 14-1, 14-3 darstellen. Über die Streifenelemente 22 wird dann jeweils eine Trägerkomponente 20 gelegt, die den Freiraum A, B bzw. 18 überspannt und mit zwei ihrer Seiten 20-1 und 20-2 über die Streifenelemente 22 ragt bzw. darüber gelegt ist vorgesehen ist (siehe Fig. 2B).

Fig. 2C zeigt beide zusammengesetzten Rahmen-Bauteile 12, 14, die jeweils entsprechend der Beschreibung von Fig. 2A und 2B zusammengesetzt wurden. Beide Rahmen-Bauteile 12, 14 werden dann zum Rahmen 16 so zusammengesetzt (siehe Fig. 2D), dass die Mittel zum Aufhängen 19, 21 sich auf gegenüber liegenden Seiten des Rahmens 16 befinden, und sich die Trägerkomponenten 20, die am Rahmen-Bauteil 12 und am Rahmen-Bauteil 14 angebracht sind, zwischen den Streifenelementen 22 im zusammengesetzten Rahmen 16 befinden.

Fig. 3A zeigt eine beispielhafte Ausführungsform einer erfindungsgemäßen zusammengesetzten Vorrichtung 10 mit einem in der Trägerkomponente gehaltenen Gewebeschnitt 26.

Fig. 3A ist ferner zu entnehmen, dass der Rahmen 16 durch Zusammenfügen der Rahmen-Bauteile 12, 14 eine Führung 28 für die Flüssigkeitszufuhr aufweist, die sich an der Seite 16-1 des Rahmens befindet. Diese Führung 28 wird durch Zusammenfügen der beiden "halben" Führungsabschnitte 28A und 28B (siehe Fig. 1A) der Rahmen-Bauteile 12, 14 gebildet.

Die erfindungsgemäße Vorrichtung 10 wird zur Kultivierung des Gewebeschnitts 26 in ein Aufnahmegefäß 30 eingeführt (siehe Fig. 3B), das in dem in Fig.3B gezeigten Beispiel ein Röhrchen mit einer Schraubkappe 31 ist. Die Vorrichtung 10 und das Aufnahmegefäß 30 bilden ein erfindungsgemäßes Kultivierungssystem 40. Das Röhrchen ist über die Schraubkappe steril belüftbar, welche bspw. einen Septum-Schlitz (nicht gezeigt) aufweist. Über die Schraubkappe 31 kann in die Führung 28 eine Perfusionsnadel 32 in das Aufnahmegefäß 30 eingeführt werden und über die Perfusionsnadel 32 Flüssigkeit zu der in dem Aufnahmegefäß 30 vorliegenden Vorrichtung 10, und damit - über die die Streifenelemente 22 und die Trägerkomponente 20 - zum Gewebeschnitt 26 geführt werden. Die zugeleitete Flüssigkeit kann dann im unteren Bereich 34 des Aufnahmegefäßes 30 aufgefangen und ggf. nach der Kultivierung analysiert werden.

In Fig. 3C ist dargestellt, wie eine kontrollierte Zuführung von Flüssigkeiten vorgenommen werden kann: Das Kultivierungssystem 40 befindet sich in einem Brutschrank 36 mit kontrollierter Atmosphäre (die Sauerstoffkonzentration wird beispielsweise durch kontrollierte Zufuhr von Stickstoff in den Brutschrank exakt reguliert). Die Perfusionsnadel 32 ist mit einem Schlauch 37 verbunden, der aus dem Brutschrank 36, der hier beispielhaft bei 37°C gehalten wird, herausgeführt ist. Der Schlauch 37 wiederum ist mit einer Spritzenpumpe 38 verbunden, durch deren Betätigen über eine Spritze kontrolliert und präzise Flüssigkeit, bspw. Kultivierungsmedium und/oder ein zu testender Wirkstoff/Medikament zugeführt werden kann. Dieses ist in dem in Fig. 3C gezeigten Beispiel bei 4°C gehalten.

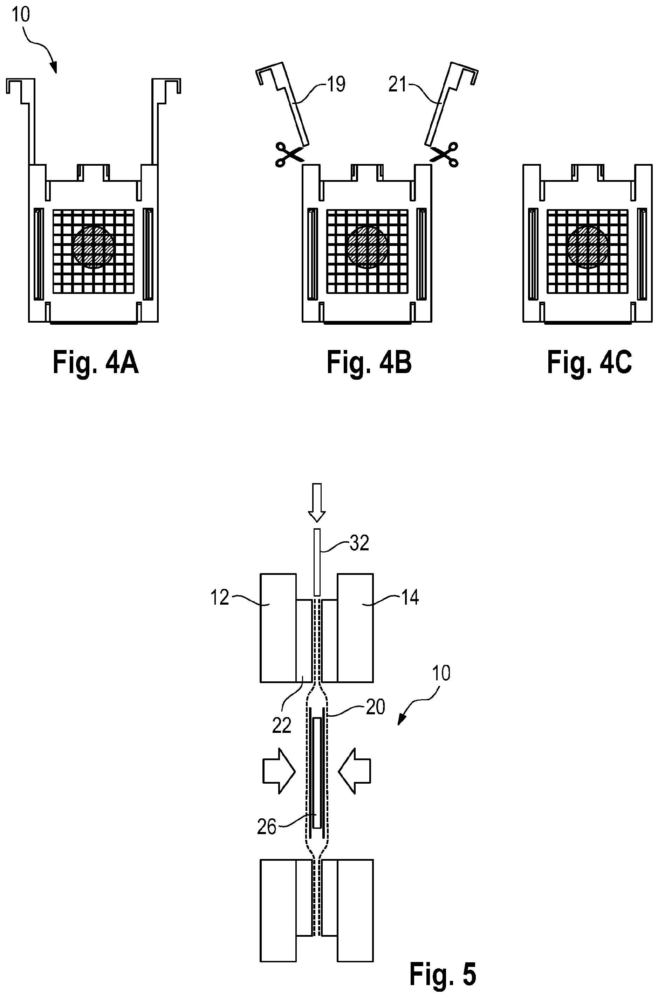

In Fig. 4 ist eine weitere vorteilhafte Handhabung der erfindungsgemäßen Vorrichtung in den Schritten A bis C gezeigt: durch ein einfaches Abschneiden der Mittel zum Aufhängen 19, 21 kann der sich in der Trägerkomponente befindliche Gewebeschnitt direkt fixiert und anschließend einfach untersucht bzw. weiteren Untersuchungen unterzogen werden. Dabei sind die gemäß einer Ausführungsform bevorzugten Maße der Rahmen mit ca. 23 x ca. 26 mm so konzipiert, dass der Rahmen nach Abschneiden der Mittel zum Aufhängen in gängige Einbett-Kassetten für Biopsien (Maße außen: L 40 x B 28 x H 6,8 mm) passt.

In Fig. 5 ist ein Längsschnitt durch die Mitte der erfindungsgemäßen Vorrichtung 10 gezeigt. Zu erkennen ist, dass die Trägerkomponente 20 im Bereich der Bauteil-Profile 13 und 15 der Rahmen-Bauteile 12, 14 zwischen den Streifenelementen 22 eingeklemmt ist. Der Gewebeschnitt 26 wiederum ist zwischen zwei Trägerkomponenten 20 im Freiraum 18 gehalten, so dass der Schnitt von beiden Seiten mit der Atmosphäre und letztendlich Sauerstoff umgeben ist. Dies bietet gegenüber herkömmlichen Kultivierungssystemen, bei denen die Gewebeschnitte insbesondere horizontal gelagert sind, den großen Vorteil, dass dem Gewebeschnitt mehr Sauerstoff zugeführt wird und dieser auch mehr Sauerstoff aufnehmen kann, was wiederum ein deutlich verbessertes Gewebe-Modell ermöglicht.

### Beispielhafte Versuchsdurchführung mit der erfindungsgemäßen Vorrichtung bzw. dem erfindungsgemäßen Kultivierungssystem

Verschiedene Ausführungsformen der erfindungsgemäßen Vorrichtung wurden hinsichtlich unterschiedlicher Trägerkomponenten getestet, hinsichtlich unterschiedlich langer Kultivierungszeiträume und hinsichtlich unterschiedlicher Gewebe/Gewebeschnitte.

Getestet wurden die gitterförmige Trägerkomponenten aus Baumwolle oder Nylon mit relativ großer Gitter-Porengröße (Baumwolle: 500 µM, Nylon: 89 µM). Ferner wurden Polycarbonat-Membranen mit einer kleinen Porengröße (12 µm) getestet sowie ein natürliches Cellulose-Hydrogel (Xellulin^{®}; Xellutec GmbH, Neuried, Deutschland)), eine Trägerkomponente aus dezellularisiertem Schweinedarm und eine Trägerkomponente aus einem 3D-gedruckten Alginat- oder Kollagen-Netz.

Sämtliche Trägerkomponenten erwiesen sich als geeignete Materialien für den Einsatz in der erfindungsgemäßen Vorrichtung (Daten teilweise nachstehend diskutiert).

Ferner wurden unterschiedliche Gewebeschnitte, die in der erfindungsgemäßen Vorrichtung bzw. dem erfindungsgemäßen Kultivierungssystem unterschiedlich lange kultiviert wurden, weiter untersucht. Hierzu wurden die erfindungsgemäßen Vorrichtungen 10, die Gewebeschnitte 26 enthielten, aus dem Aufnahmegefäß 30 entfernt, die Mittel zum Aufhängen 19, 21 abgeschnitten und die Vorrichtungen 10, die die unterschiedlichen Gewebeschnitte enthielten, in kommerziell verfügbare Einbett-Kassetten für die Immunfärbung hinsichtlich bestimmter Marker eingelegt.

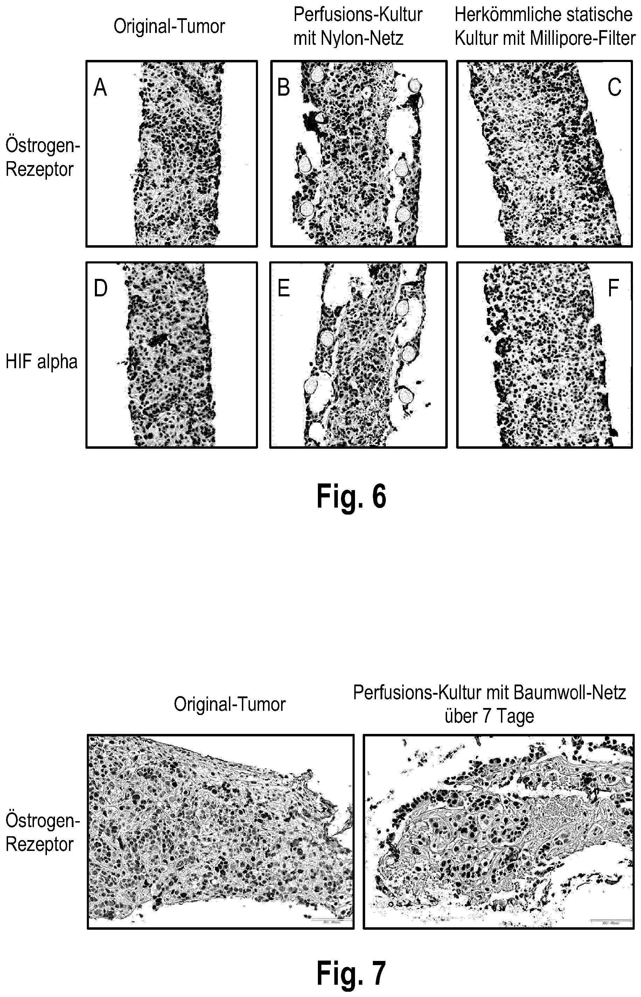

Anhand der nachstehend diskutierten Immunfärbungen zeigte sich die praktische Handhabung der erfindungsgemäßen Vorrichtung mit den Einbett-Kassetten. Schnitte eines original Tumorgewebes wurden hinsichtlich der Marker Östrogen-Rezeptor und Hypoxie-induzierbarer Faktor 1 (HIF1) alpha immunhistochemisch gefärbt. Im Vergleich dazu wurden Gewebeschnitte des gleichen Tumors einmal mit dem erfindungsgemäßen Kultivierungssystem jeweils drei Tage lang kultiviert, und zwar mit einem Nylon-Netz, und andererseits mit einem herkömmlichen Filter (Millicell^{®} Cell Culture Inserts; Merck Millipore, PTFE, Porengröße 0,4 µm). Die Ergebnisse dieser Kultivierungsversuche sind in Fig. 6 dargestellt, wonach sich zeigte, dass die Gewebeschnitte, die mittels des erfindungsgemäßen Kultivierungssystems kultiviert wurden (Fig. 6B, E), eine ähnliche Morphologie und Biomarker-Expression aufwiesen wie der Original-Tumor-Gewebeschnitt (Fig. 6A, D). Hingegen zeigte der Tumor-Gewebeschnitt, der mittels der herkömmlichen Vorrichtung kultiviert wurde (Fig. 6C, F), hinsichtlich der Expression der Tumor-Biomarker Östrogen-Rezeptor und HIF1-alpha einen künstlichen Gradienten von der Atmosphären-Seite zu der Filter-Seite, was zu einem unausgeglichenen Zellkultur-Modell führt, das letztendlich für verlässliche in vitro-Tests von Medikamenten nicht so geeignet ist wie die erfindungsgemäße Vorrichtung.

Ferner wurde in weiteren Versuchen gezeigt, dass die erfindungsgemäße Vorrichtung auch für längere Kultivierungsversuche eingesetzt werden kann. Die Ergebnisse dieser Versuchsreihen sind in Fig. 7 gezeigt: Auch nach einer 7-tägigen Kultivierungsdauer mit dem erfindungsgemäßen System (rechts in Fig. 7) zeigte der Tumor-Gewebeschnitt eine ähnliche Morphologie und Biomarker-Expression wie der nicht-kultivierte Original-Tumor (links in Fig. 7), was anhand der Färbungen hinsichtlich des Östrogen-Rezeptors nachgewiesen wurde.

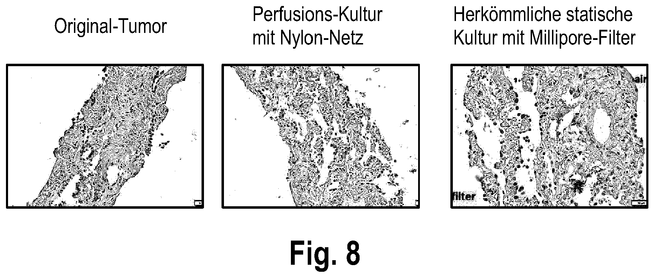

Schließlich wurden auch Primärgewebeschnitte von einem Patienten mit Lungenkrebs vergleichend kultiviert und immunhistochemisch gefärbt. Diese Ergebnisse sind in Fig. 8 gezeigt: Links ist die Immunfärbung auf HIF1-alpha für den Original-Tumor gezeigt, in der Mitte die Immunfärbung des mit dem erfindungsgemäßen System auf einem Nylon-Netz 5 Tage lang kultivierten Schnitts des gleichen Tumors, und rechts die Immunfärbung eines Schnitts des gleichen Tumors, der 5 Tage mit einem herkömmlichen System (Millipore) kultiviert wurde. Es zeigte sich, dass der Gewebeschnitt, der mit dem erfindungsgemäßen System kultiviert wurde, die gleiche Morphologie und HIF1-alpha-Expression zeigte wie der Original-Tumorgewebeschnitt, wohingegen der auf dem herkömmlichen Filter kultivierte Gewebeschnitt wieder einen Gradienten von der Atmosphären-Seite zur Filter-Seite zeigte.

Die oben geschilderten Versuche belegen eindrucksvoll den deutlichen Vorteil, den das erfindungsgemäße Kultivierungssystem/die erfindungsgemäße Vorrichtung gegenüber den herkömmlichen Kultivierungsvorrichtungen und -methoden von Geweben/Gewebeschnitten bietet.

Mit den erfindungsgemäßen Systemen und Vorrichtungen wird es damit ermöglicht, die Wirkung von Medikamenten auf Tumor-Gewebe auch über längere Zeiträume in einem reproduzierbaren und mit dem Original-Gewebe vergleichbaren Modell zu testen.

## Patentansprüche

1. Vorrichtung (10) zur Kultivierung von Gewebeschnitten (26), insbesondere für die Testung von Wirkstoffen und/oder Medikamenten, wobei die Vorrichtung (10) Folgendes aufweist:
einen Rahmen (16) mit einem umlaufenden Profil (17) und einen vom umlaufenden Profil gebildeten mittigen Freiraum (18),
zumindest eine Trägerkomponente (20), wobei die Trägerkomponente (20) zur Aufnahme eines Gewebeschnittes (26) ausgestaltet ist, und
zumindest ein Flüssigkeits-absorbierendes Streifenelement (22),
**dadurch gekennzeichnet, dass** der Rahmen (16) zum Halten der zumindest einen Trägerkomponente (20) und des zumindest einen Streifenelements (22) ausgestaltet ist, derart, dass das zumindest eine Streifenelement (22) im Bereich des umlaufenden Profils (17) angebracht ist, und dass die zumindest eine Trägerkomponente (20) den Freiraum (18) überspannend und das zumindest eine Streifenelement (22) überlappend am Rahmen (16) gehalten wird.

2. Vorrichtung (10) nach Anspruch 1, **dadurch gekennzeichnet, dass** der Rahmen (16) zwei Rahmen-Bauteile (12; 14) mit jeweils einem umlaufenden Bauteil-Profil (13; 15) und einem vom umlaufenden Bauteil-Profil (13; 15) gebildeten jeweils mittigen Bauteil-Freiraum (A; B) umfasst, welche Rahmen-Bauteile (12; 14) mittels eines Verbindungselements deckend zum Rahmen zusammensetzbar sind, wobei das Verbindungselement vorzugsweise als Klickverbindung, Führungsschiene, Klemmverbindung oder als Verschluss ausgestaltet ist.

3. Vorrichtung (10) nach einem der vorstehenden Ansprüche, **dadurch gekennzeichnet, dass** der Rahmen (16) eine Führung (28) zur Flüssigkeitszufuhr aufweist.

4. Vorrichtung (10) nach einem der vorstehenden Ansprüche, **dadurch gekennzeichnet, dass** der Rahmen (16) und/oder die zwei Rahmen-Bauteile (12; 14) Mittel (24) zur Befestigung des zumindest einen Streifenelements (22) aufweist.

5. Vorrichtung (10) nach einem der vorstehenden Ansprüche, **dadurch gekennzeichnet, dass** der Rahmen (16) und/oder die zwei Rahmen-Bauteile (12; 14) Mittel zur Aufhängung (19; 21) der Vorrichtung (10) aufweist.

6. Vorrichtung (10) nach einem der vorstehenden Ansprüche, **dadurch gekennzeichnet, dass** das zumindest eine Streifenelement (22) als blattförmiges Fließpapier ausgestaltet ist.

7. Vorrichtung (10) nach einem der vorstehenden Ansprüche, **dadurch gekennzeichnet, dass** die zumindest eine Trägerkomponente (20) aus einem Material, ausgewählt aus der Gruppe bestehend aus Baumwollgewebe, Nylongewebe, Polycarbonat, Zellulose-Hydrogel, Tierdarm, insbesondere Schweinedarm, 3D-gedruckte Biomaterialien, insbesondere 3D-gedrucktes Alginatgewebe oder 3D-gedrucktes Collagengewebe, ausgestaltet ist.

8. Vorrichtung (10) nach einem der vorstehenden Ansprüche, **dadurch gekennzeichnet, dass** der Rahmen (16) aus einem lösungsmittelbeständigen Material ausgestaltet ist.

9. Vorrichtung (10) nach einem der Ansprüche 2 bis 8, **dadurch gekennzeichnet, dass** jedes der zwei Rahmen-Bauteile (12; 14) jeweils zwei Streifenelemente (22) aufweist, die jeweils an gegenüber liegenden Seiten (12-1, 12-3; 14-1, 14-3) eines Bauteil-Profils (13; 15) angebracht sind, sowie jeweils eine Trägerkomponente (20), die den jeweiligen Bauteil-Freiraum (A; B) überspannend und die zwei Streifenelemente (22) überlappend an jeweils einem Rahmen-Bauteil (12; 14) gehalten wird.

10. Kultivierungssystem (40) zur Kultivierung von Gewebeschnitten (26), insbesondere für die Testung von Wirkstoffen und/oder Medikamenten, **dadurch gekennzeichnet, dass** das Kultivierungssystem (40) eine Vorrichtung (10) gemäß einem der Ansprüche 1 bis 9 und ein Aufnahmegefäß (30) aufweist, wobei die Vorrichtung (10) in das Aufnahmegefäß (30) vertikal hängend einbringbar ist.

11. Kultivierungssystem (40) nach Anspruch 10, **dadurch gekennzeichnet, dass** das Aufnahmegefäß (30) ein belüftbares Röhrchen ist.

12. Verwendung der Vorrichtung (10) nach einem der Ansprüche 1 bis 9, oder des Kultivierungssystems (40) nach Anspruch 10 oder 11, zur Kultivierung von Gewebeschnitten (26) für die Testung von Wirkstoffen und/oder Medikamenten.

## Claims

1. Device (10) for the culturing of tissue sections (26), especially for the testing of active ingredients and/or medicaments, the device (10) having the following:
a frame (16) with an enclosing profile (17) and a central free space (18) formed by the enclosing profile,
at least one support component (20), the support component (20) being configured to accommodate a tissue section (26), and
at least one liquid-absorbing strip element (22),
**characterized in that** the frame (16) is configured to hold the at least one support component (20) and the at least one strip element (22) such that the at least one strip element (22) is mounted in the region of the enclosing profile (17) and that the at least one support component (20) is held on the frame (16) with spanning of the free space (18) and overlapping of the at least one strip element (22).

2. Device (10) according to Claim 1, **characterized in that** the frame (16) comprises two frame members (12; 14), each with an enclosing member profile (13; 15) and a central member free space (A; B) formed in each case by the enclosing member profile (13; 15), which frame members (12; 14) are assemblable in a covering manner to form the frame by means of a connecting element, the connecting element preferably being configured as a click connection, guide rails, clamp connection or as a clasp.

3. Device (10) according to either of the preceding claims, **characterized in that** the frame (16) has a duct (28) for the supply of liquid.

4. Device (10) according to any of the preceding claims, **characterized in that** the frame (16) and/or the two frame members (12; 14) has means (24) for fixing of the at least one strip element (22).

5. Device (10) according to any of the preceding claims, **characterized in that** the frame (16) and/or the two frame members (12; 14) has means for hanging (19; 21) of the device (10).

6. Device (10) according to any of the preceding claims, **characterized in that** the at least one strip element (22) is configured as sheet-shaped blotting paper.

7. Device (10) according to any of the preceding claims, **characterized in that** the at least one support component (20) is configured from a material selected from the group consisting of cotton fabric, nylon fabric, polycarbonate, cellulose hydrogel, animal intestine, especially pig intestine, 3D-printed biomaterials, especially 3D-printed alginate fabric or 3D-printed collagen fabric.

8. Device (10) according to any of the preceding claims, **characterized in that** the frame (16) is configured from a solvent-resistant material.

9. Device (10) according to any of Claims 2 to 8, **characterized in that** each of the two frame members (12; 14) has respectively two strip elements (22) which are each mounted on opposite sides (12-1, 12-3; 14-1, 14-3) of a member profile (13; 15) and has respectively one support component (20) which is held on a frame member (12; 14) in each case with spanning of the respective member free space (A; B) and overlapping of the two strip elements (22).

10. Culturing system (40) for the culturing of tissue sections (26), especially for the testing of active ingredients and/or medicaments, **characterized in that** the culturing system (40) has a device (10) according to any of Claims 1 to 9 and an accommodation vessel (30), the device (10) being introducible into the accommodation vessel (30) in a vertically hanging manner.

11. Culturing system (40) according to Claim 10, **characterized in that** the accommodation vessel (30) is a ventilatable tube.

12. Use of the device (10) according to any of Claims 1 to 9, or of the culturing system (40) according to Claim 10 or 11, for the culturing of tissue sections (26) for the testing of active ingredients and/or medicaments.

## Revendications

1. Dispositif (10) destiné à la culture de coupes de tissu (26), en particulier pour tester des substances actives et/ou des médicaments, le dispositif (10) comprenant ce qui suit :
un cadre (16) comportant un profil périphérique (17) et un espace libre central (18) formé par le profil périphérique,
au moins un composant support (20), le composant support (20) étant conçu pour recevoir une coupe de tissu (26), et
au moins un élément en forme de bande absorbant les liquides (22),
**caractérisé en ce que** le cadre (16) est conçu pour maintenir ledit au moins un composant support (20) et ledit au moins un élément en forme de bande (22), de telle sorte que ledit au moins un élément en forme de bande (22) est fixé dans la région du profil périphérique (17), et que ledit au moins un composant support (20) est maintenu sur le cadre (16) en recouvrant l'espace libre (18) et en chevauchant ledit au moins un élément en forme de bande (22).

2. Dispositif (10) selon la revendication 1, **caractérisé en ce que** le cadre (16) comprend deux éléments de cadre (12 ; 14) comportant chacun un profil périphérique d'élément (13 ; 15) et un espace libre central d'élément (A ; B) formé par le profil périphérique d'élément (13 ; 15), lesquels éléments de cadre (12 ; 14) peuvent être assemblés de manière superposée pour former le cadre au moyen d'un élément de liaison, l'élément de liaison étant de préférence conçu sous la forme d'une liaison par encliquetage, d'un rail de guidage, d'une liaison par serrage ou d'un dispositif de fermeture.

3. Dispositif (10) selon l'une quelconque des revendications précédentes, **caractérisé en ce que** le cadre (16) comporte un guide (28) destiné à l'alimentation en liquide.

4. Dispositif (10) selon l'une quelconque des revendications précédentes, **caractérisé en ce que** le cadre (16) et/ou les deux éléments de cadre (12 ; 14) comportent des moyens (24) destinés à fixer ledit au moins un élément en forme de bande (22).

5. Dispositif (10) selon l'une quelconque des revendications précédentes, **caractérisé en ce que** le cadre (16) et/ou les deux éléments de cadre (12 ; 14) comportent des moyens de suspension (19 ; 21) du dispositif (10).

6. Dispositif (10) selon l'une quelconque des revendications précédentes, **caractérisé en ce que** ledit au moins un élément en forme de bande (22) est conçu sous la forme d'un papier buvard en feuille.

7. Dispositif (10) selon l'une quelconque des revendications précédentes, **caractérisé en ce que** ledit au moins un composant support (20) est constitué d'un matériau choisi dans le groupe constitué par un tissu de coton, un tissu de nylon, du polycarbonate, un hydrogel de cellulose, un intestin animal, en particulier un intestin de porc, des biomatériaux imprimés en 3D, en particulier un tissu d'alginate imprimé en 3D ou un tissu de collagène imprimé en 3D.

8. Dispositif (10) selon l'une quelconque des revendications précédentes, **caractérisé en ce que** le cadre (16) est constitué d'un matériau résistant aux solvants.

9. Dispositif (10) selon l'une quelconque des revendications 2 à 8, **caractérisé en ce que** chacun des deux éléments de cadre (12 ; 14) comporte deux éléments en forme de bande (22), qui sont fixés respectivement sur des côtés opposés (12-1, 12-3 ; 14-1, 14-3) d'un profil d'élément (13 ; 15), ainsi qu'un composant support (20), qui est maintenu sur l'élément de cadre respectif (12 ; 14) en recouvrant l'espace libre d'élément respectif (A ; B) et en chevauchant les deux éléments en forme de bande (22).

10. Système de culture (40) destiné à la culture de coupes de tissu (26), en particulier pour tester des substances actives et/ou des médicaments, **caractérisé en ce que** le système de culture (40) comprend un dispositif (10) selon l'une quelconque des revendications 1 à 9 et un récipient de réception (30), le dispositif (10) pouvant être introduit dans le récipient de réception (30) en étant suspendu verticalement.

11. Système de culture (40) selon la revendication 10, **caractérisé en ce que** le récipient de réception (30) est un tube pouvant être aéré.

12. Utilisation du dispositif (10) selon l'une quelconque des revendications 1 à 9 ou du système de culture (40) selon la revendication 10 ou 11 pour la culture de coupes de tissu (26) destinées à tester des substances actives et/ou des médicaments.
